(19)

**Europäisches Patentamt**

**European Patent Office**

**Office européen des brevets**

(11)  **EP 4 296 951 A1**

(12)  **EUROPEAN PATENT APPLICATION**

(43) Date of publication:
**27.12.2023   Bulletin 2023/52**

(21) Application number: **22305905.6**

(22) Date of filing: **22.06.2022**

(51) International Patent Classification (IPC):
**G06T 7/77** *(2017.01)*          **G06T 7/00** *(2017.01)*
**G06T 7/30** *(2017.01)*

(52) Cooperative Patent Classification (CPC):
**G06T 7/77; G06T 7/0012; G06T 7/30;**
G06T 2207/30016; G06T 2207/30096

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **Assistance Publique Hôpitaux de Paris**
  **75004 Paris (FR)**
• **Université Paris Cité**
  **75006 Paris (FR)**
• **Ecole Polytechnique**
  **91120 Palaiseau (FR)**
• **Institut National de Recherche en Informatique et
  en Automatique**
  **78150 Le Chesnay-Rocquencourt (FR)**

(72) Inventors:
• **BOEKEN, TOM**
  **75016 PARIS (FR)**
• **DEBAVELAERE, Vianney**
  **92220 BAGNEUX (FR)**
• **FEYDY, Jean**
  **94340 JOINVILLE LE PONT (FR)**
• **ALLASSONNIERE, Stéphanie**
  **92340 BOURG LA REINE (FR)**
• **PELLERIN, Olivier**
  **75016 PARIS (FR)**
• **SAPOVAL, MARC**
  **94110 ARCUEIL (FR)**

(74) Representative: **Plasseraud IP**
  **66, rue de la Chaussée d'Antin**
  **75440 Paris Cedex 09 (FR)**

(54)  **METHOD IMPLEMENTED BY COMPUTER MEANS FOR CHARACTERIZING AT LEAST ONE
OBSERVATION OF A SUBJECT**

(57)    The invention relates to a method implemented by computer means for characterizing at least one observation y of a subject, comprising the steps of determining a template $\bar{y}$ characterizing a population of subjects without anomaly, by diffeomorphic deformation, minimizing the cost function J :

$$J(F,A) = K\|y - F(\bar{y}) - A\|^2 + \lambda\|A\|_1 + Reg(F)$$

where F is a deformation function, A is an anomaly matrix, $\|A\|_1$ is the 1-norm of *A, K* and $\lambda$ are predefined constants, Reg is a regularization function,
where during minimization, F and A are determined by learning, F providing information on the morphological variability of the subject and A providing information on anomalies in said observation y.

**EP 4 296 951 A1**

**Description**

The present invention relates to a method implemented by computer means for characterizing at least one observation of a subject. Said observation may be an image, for example a medical image, or a feature of an image. Said subject may be a patient or an area of a patient, for example an organ of said patient.

It is known to apply deep learning techniques in order to characterize medical images, for example in order to detect anomalies such as lesions or tumors in said images.

Such techniques traditionally consist in training a model from training data comprising images and annotations indicating if and which anomalies appear on each image. This trained model is then used during inference to characterize new images, which are not present in the training set.

The main disadvantage of these techniques is that they require a large number of images to compose the training set, and the anomalies must be labelled or labelled by doctors for each image. Such techniques may also be prone to overfitting, which means that the results obtained with the trained model fit too closely to the training data but fail to generalize on new data, i.e. fail to reliably predict anomalies on new images.

The present invention proposes a method that does not require any annotation or label nor a large training set and can straightforwardly be generalized to many applications.

To that end, the invention proposes a method implemented by computer means for characterizing at least one observation y of a subject, comprising the steps of:

- determining a template $\bar{y}$ characterizing a population of subjects without anomaly, by diffeomorphic deformation,

- minimizing the following cost function $J$:

$$J(F,A) = K\|y - F(\bar{y}) - A\|^2 + \lambda\|A\|_1 + Reg(F)$$

where:

F is a deformation function,

$A$ is an anomaly matrix,

$\|A\|_1$ is the 1-norm *of A,*

K and $\lambda$ are constant values,

*Reg* is a regularization function,

wherein during minimization, F and A are determined by learning, F providing information on the morphological variability of the subject and A providing information on anomalies in said observation y.

Said observation may be an image. Said image may be a 2-dimensionnal or a 3-dimensionnal image.

Said observation may be a feature of an image, for example a contour. Said contour may be an open or a closed contour.

Said image may be a medical image, for example an MRI image of an area of a subject, in this case a patient. Said area may be an organ of said patient.

Said image may be a non-medical image. For example, said image may illustrate variations in time of a given feature (temperature for material cooking, etc..).

The template $\bar{y}$ may be obtained par Large Diffeomorphic Deformation Metric Mapping or LDDMM.

The LDDMM framework is known from the following articles:

- Alain Trouve. An infinite dimensional group approach for physics based models in pattern recognition. preprint, 1995.
- Paul Dupuis, Ulf Grenander, and Michael I Miller. Variational problems on flows of diffeomorphisms for image matching. Quarterly of applied mathematics, pages 587-600, 1998.
- M Faisal Beg, Michael I Miller, Alain Trouve, and Laurent Younes. Computing large deformation metric mappings via geodesic flows of diffeomorphisms. International journal of computer vision, 61(2):139-157, 2005.

The creation of a template using the LDDMM framework is also known from the article "Construction of Bayesian deformable models via stochastic approximation algorithm: A convergence study. S. Allassonnière, E. Kuhn, A. Trouvé. Bernoulli Journal, Vol 16(3), p.641-678, 2010."

The template $\bar{y}$ may be based on a plurality of observations, for example images, that do not contain anomalies, called control observations.

A determination method of such template $\bar{y}$ using an LDDMM framework and based on a plurality of images is explained in detail below.

Given a dataset $(y_i)_{1 \leq i \leq n}$ of images of dimension $d \in \{2,3\}$, the aim of said method is to create a template $\bar{y}$, i.e. a representative image of a population of subjects.

To do so, a distance between observations or images using diffeomorphic deformations is created.

Let $V$ be a Reproducible Kernel Hilbert Space. For $x \in \mathbb{R}^n$, a vector field v is represented as:

$$v(x) = \sum_{i=1}^{n_{cp}} K_V(c_i, x)\alpha_i$$

(E1)

where $(c_i)_{1 \leq i \leq n_{cp}}$ are called control points and $(\alpha_i)_{1 \leq i \leq n_{cp}}$ are called momenta. v is thus represented as the interpolation of the momenta at the control points using the kernel $K_V$. In practice, $K_V$ is chosen to be a Gaussian kernel with variance $\sigma_V^2$: for $x, y \in \mathbb{R}^n$,

$$K_V(x,y) = \exp\left(-\frac{\|x-y\|^2}{2\,\sigma_V^2}\right).$$

Let $\mathbb{L}^2([0,1], V) = \left\{(v_t)_{t \in [0,1]} \,\middle|\, \forall t \in [0,1], v_t \in V \text{ and } \int_0^1 \|v_t\|_V^2 \, dt < \infty\right\}$.

Given $v \in \mathbb{L}^2([0,1], V)$, we set $\phi_1^v$ the diffeomorphism obtained as the flow at time 1 of the vector field $v$:

$$\begin{cases} \partial_t \phi_t^v = v_t \circ \phi_t^v \\ \phi_0^v = \text{Id}. \end{cases}$$

(E1)

We then set $G = \{\phi_1^v \mid v \in \mathbb{L}^2([0,1], V)\}$ the group of such diffeomorphisms.

It is now possible to define a distance on G. For $\phi, \phi' \in G$, we set:

$$d_G(Id, \phi) = \inf\left\{\left(\int_0^1 \|v_t\|_V^2 \, d_t\right)^{1/2} \,\middle|\, v \in \mathbb{L}^2([0,1], V) \text{ and } \phi_1^v = \phi\right\}$$

and

$$d_G(\phi, \phi') = d_G(Id, \phi' \circ \phi^{-1}).$$

This states that G is given the structure of a manifold on which distances are computed as the length of minimal

geodesic paths $(\phi_t^v)_{t\in[0,1]}$ connecting two elements.

It has been showed that this infimum is a minimum and that the distance is right invariant. Moreover, a geodesic in G passing through *Id* at the initial time is then uniquely defined by an initial velocity $v_0$, i.e. by initial control points and momenta.

In the following, we write $\varepsilon xp_t(v_0)$ the value of this geodesic at the time t.

Hence, for two shapes *x* and *y* $\in$ *M*, we set:

$$d(x,y) = inf \left\{ \left( \int_0^1 \|v_t\|^2 \, d_t \right)^{1/2} \mid v \in \mathbb{L}^2([0,1], V) \text{ and } \phi_1^v . x = y \right\}.$$

This distance measures the shortest length of the path relying x to y using the diffeomorphisms $\phi^v$. It also allows to define a Riemannian structure on M. A geodesic on M will then be defined using an initial shape $p_0$ and initial velocity $v_0$ by $t \mapsto \varepsilon xp_t(v_0)(p_0)$.

Hence, we measure the distance between two shapes as the difficulty to deform one onto another. Moreover, as $\phi_1^v$ is a diffeomorphism, it is invertible and preserves the smoothness and structure of the shapes.

We use those geodesics to define a template of the data set, as well as the deformations from this template towards each subject using inexact matching. More precisely, we set the following cost function:

$$J(\bar{y}, c_0, \alpha_i) = \frac{1}{2\sigma^2} \sum_{i=1}^n \left\| y_i - \varepsilon xp_t(v_i).\bar{y} \right\|_2^2 + \frac{1}{2} \|v_i\|_V^2 \, ,$$

where $v_i$ is obtained using the control points $c_0$ and momenta $\alpha_i$.

$\bar{y}$ is then the template of the population and can be estimated by minimizing said cost function using usual gradient descent algorithms for example. Other algorithms may also be used.

Once such template or control template $\bar{y}$ based on a plurality of images that do not contain anomalies has been obtained, the following steps or model may be applied.

This model aims at highlighting the anomalies of subjects with respect to said control template. As anomalies are modeled, we make the assumption that these anomalies are sparse in the corresponding area of the subject.

Apart from these anomalies, the rest of the area may be similar to the control template. Therefore, the following model is proposed.

We write $(y_i)_{1\le i\le n}$ the observations of *n* subjects and $\bar{y} \in \mathbb{R}^d$ the control template obtained using the above-mentioned method. We suppose that the images all have the same size $\prod_{j=1}^d n_i$ where d = 2 or 3 for 2d or 3d images.

Each observation $y_i$ can be written as:

$$y_i = \varepsilon xp_1(v_i)(\bar{y}) + A_i + \varepsilon_i$$

This equation means that the observation $y_i$ of each subject is obtained as the diffeomorphic deformation of the template $\bar{y} : \varepsilon xp_1(v_i)(\bar{y})$ to which a matrix $A_i$ containing the anomalies of said observation and a noise $\varepsilon_i$ are added.

As explained above, $v_i$ is a velocity field obtained as the interpolation of momenta $\alpha_i \in (\mathbb{R}^d)^{n_{cp}}$ at $n_{cp} \in$ N control points $c_i \in (\mathbb{R}^d)^{n_{cp}}$ using a Gaussian kernel as follows: $\forall x \in \mathbb{R}^d$ ;

$$v_i(x) = \sum_{j=1}^{n_{cp}} \exp\left( -\frac{\left\| c_{i,j} - x \right\|^2}{2\sigma^2} \right) \alpha_{i,j} .$$

(E3)

In order to enforce the expected sparsity of the anomalies, a $L^1$ regularization on $A_i$ is added. This is also a way to prevent it from including the noise, modeled as following a centered normal distribution.

Given this model, the goal is to estimate jointly the deformations from the given template and the anomaly matrices for each observation. Here, as the control template has already been estimated, each observation can be processed separately, and the following function is minimized:

$$J_i(c_i, \alpha_i, A_i) = \frac{1}{2\sigma^2}\left|\left|y_i - \varepsilon\exp_1(v_i)(\bar{y}) - A_i\right|\right|_2^2 + \lambda\left|\left|A_i\right|\right|_1 + \frac{1}{2}\left|\left|v_i\right|\right|_V^2,$$

$$(E4)$$

where $v_i$ is obtained using equation (E3). The first term of $J_i$ measures the distance between the observation $y_i$ and the reconstruction $\varepsilon\mathrm{xp}_1(v_i)(\bar{y}) + A_i$ while the other terms $\lambda\|A_i\|_1$ and $\frac{1}{2}\left|\left|v_i\right|\right|_V^2$ respectively measure the sparsity of $A_i$ and the regularity of the diffeomorphic deformation.

To minimize $A_i$, as $\|.\|_1$ is not differentiable, a proximal gradient descent algorithm may be implemented, using for example the Pytorch package to automatically compute the gradients of the differentiable part of $J_i$.

Alternatively, the template $\bar{y}$ may be based on only one observation that do not contain anomalies, also called control observation, and at least one observation that does contain anomalies.

Indeed, in some cases, it can be difficult to obtain a dataset of control observations, necessary to create the control template $\bar{y}$. It is for instance the case for brains where we barely get a MRI scan from a control patient.

In that case, the template $\bar{y}$ may be created along the estimation of the anomaly matrices using only the image of one control patient $y_0$. To do so, we consider $\bar{y}$ to be the diffeomorphic deformation of a control hypertemplate $y_0$:

$$\bar{y} = \varepsilon\exp_1(v_0)(y_0),$$

$$(E5)$$

where $v_0$ is obtained as the interpolation of momenta $\alpha_0$ at control points $c_0$, as explained above.

As $y_0$ is a control observation, it has no anomaly, and its diffeomorphic deformation $\bar{y}$ has no anomaly either.

Hence, in that case, we not only estimate the velocities $(v_i)_{1 \leq i \leq n}$ and sparse matrices $(A_i)_{1 \leq i \leq n}$ but also the velocity $v_0$ by minimizing:

$$\tilde{J}(c_0, (\alpha_i)_{0 \leq i \leq n}, (A_i)_{1 \leq i \leq n}) = \sum_{i=1}^{n}\left(\frac{1}{2\sigma^2}\left|\left|y_i - \varepsilon\exp_1(v_i)(\bar{y}) - A_i\right|\right|_2^2 + \lambda\left|\left|A_i\right|\right|_1 + \frac{1}{2}\left|\left|v_i\right|\right|_V^2\right) + \frac{1}{2}\left|\left|v_0\right|\right|_V^2,$$

$$(E6)$$

where $\bar{y}$ is obtained using the velocity $v_0$ and $v_i$ is obtained as the interpolation of the momenta $\alpha_i$ at the control points $c_0$.

Note that, as the template is a reference image for the whole population, the energy to minimize is not separable and involves the contributions of all subjects.

Once again, this optimization may be done by proximal gradient descent.

The same control points $c_0$ may be used for all velocity fields $(v_i)_{1 \leq i \leq n}$ in order to reduce the computation time. In alternative, the control points $c_i$ may differ with the velocity fields $(v_i)_{1 \leq i \leq n}$.

In practice, computational problem may sometimes occur when estimating the parameters of those models. Indeed, the gradient descent may tend to stay blocked in local minima. By including the errors of reconstruction in the anomaly matrix, the algorithm often chooses not to improve the reconstruction and stays blocked in a local minimum. To solve this problem, the anomaly matrix may be prevented to take any value outside of the reconstruction of the object for the first iterations, for example for the first 100 iterations. This allows to improve the diffeomorphic reconstruction and to reach a more relevant area of interest of the energy landscape.

In the following, the above-mentioned model may be compared to the usual cross-sectional atlas, estimating the template $\bar{y}$ and the deformations towards the observations without the use of anomaly matrices nor hypertemplate.

This writes as minimizing the following energy:

$$J_0(\bar{y}, c_0, (\alpha_i)_{1 \le i \le n}) = \frac{1}{2\sigma^2}\sum_{i=1}^{n}\left\|y_i - \varepsilon\exp_1(v_i)(\bar{y})\right\|_2^2 + \frac{1}{2}\sum_{i=1}^{n}\left\|v_i\right\|_V^2 \, .$$

(E7)

The above-mentioned model may also be compared the cross-sectional atlas when the template is obtained via a control hypertemplate. As above, $v_0$ is obtained as the interpolation of momenta $\alpha_0$ at control points $c_0$. The functional to minimize is now:

$$J_1(c_0, (\alpha_i)_{0 \le i \le n}) = \frac{1}{2\sigma^2}\sum_{i=1}^{n}\left\|y_i - \varepsilon\exp_1(v_i)(\bar{y})\right\|_2^2 + \frac{1}{2}\sum_{i=0}^{n}\left\|v_i\right\|_V^2 \, .$$

(E8)

where $\bar{y}$ is defined by equation E5.

The estimation of those two models can be done using a usual gradient descent.

The invention also proposes a computer software, comprising instructions to implement at least a part of the above-mentioned method when the software is executed by a processor.

The invention also proposes a computer device comprising:

- an input interface to receive at least one observation y of a subject,

- a memory for storing at least instructions of above-mentioned computer program,

- a processor accessing to the memory for reading the aforesaid instructions and executing then the above-mentioned method,

- an output interface to provide F and A determined during minimization of the cost function J.

The invention also proposes a computer-readable non-transient recording medium on which a computer software is registered to implement the above-mentioned method, when the computer software is executed by a processor.

Other features, details and advantages will be shown in the following detailed description and on the figures, on which:

- figure 1 shows schematically an example of a computer device according to the invention;
- figure 2 shows the comparison of the methods according to the invention with regard to other known methods ;
- figure 3 also shows the comparison of the methods according to the invention with regard to another method;
- figure 4 shows a template estimated using a control patient as a hypertemplate;
- figure 5 shows results for four different subjects;
- figure 6 shows a patient observation and the anomaly matrix in a case where the tumour is not detected;
- figure 7 shows patient observations and estimated anomaly matrices;
- figure 8 shows initial observations of livers and said images after convolution processing;
- figure 9 shows a template estimated as a diffeomorphic deformation of a control patient;
- figure 10 shows an observation, a reconstruction and the corresponding anomaly matrix;
- figure 11 shows an observation, the corresponding anomaly matrix and the negative value of said matrix;
- figure 12 illustrates the importance to apply a Gaussian convolution to the data set before estimating the parameters of the model;

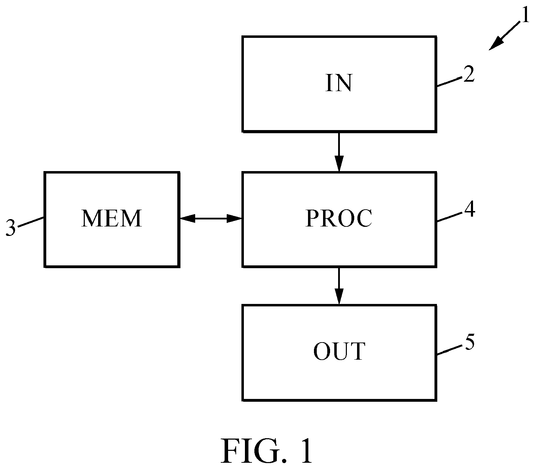

Figure 1 schematically shows an example of a computer device 1 according to the invention. Said computer device 1 comprises:

- an input interface 2,

- a memory 3 for storing at least instructions of a computer program,

- a processor 4 accessing to the memory 3 for reading the aforesaid instructions and executing the method or algorithm according to the invention,

- an output interface 5.

The method or algorithm implemented according to the invention will now be described through several examples.

Example 1

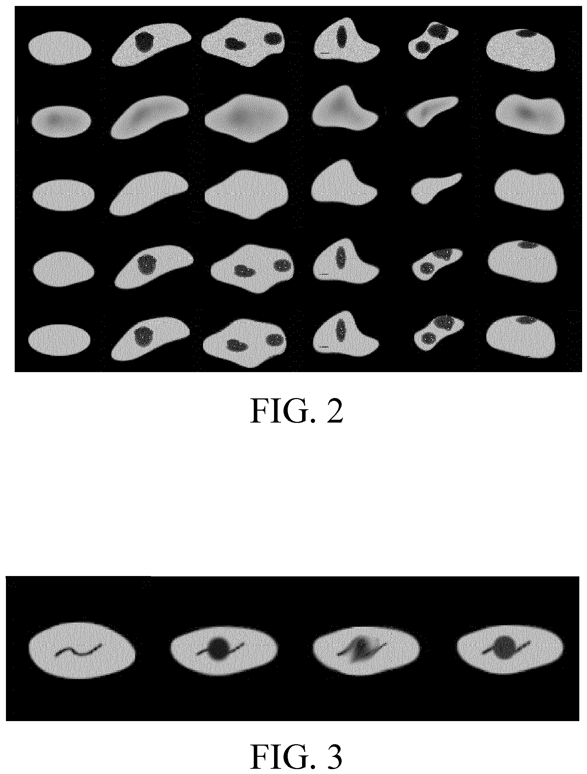

To test the above-mentioned model, a simulated data set of 500 observations deformed from a common template has been created, to which a random number of dark spots (between 1 and 5) and some Gaussian noise have been added. This template is created as the deformation of an ellipse (hypertemplate). A "control" data set of 100 observations of subjects has also been created, without dark spot from the same template, to be able to estimate a control template $\bar{y}$ and use it in the case of the model (E4).
The template and five observations with dark spots are presented on the first line of Figure 2.
More particularly, this figure shows:

- on the top line, the template and five observations,

- on the second line, the estimated template and reconstructed subjects without the use of hypertemplate nor anomaly matrix (model (E7)),

- on the third line, the results when one uses the hypertemplate but no anomaly matrix (model (E0)),

- on the fourth line, we first estimate a template (first column) from control subjects and then reconstruct the other subjects using an anomaly matrix (model (E4))

- on the last line, the results with sparse matrices when the estimation of a template is done at the same time, from a hypertemplate (model (E6))

We first apply the model where the template is directly estimated, without the use of a hyper-template nor sparse matrices (E7).
As can be seen on the second line of Figure 2, a dark shadow is created on the estimated template. Similarly, this dark shadow is reported on the reconstructions of each observation. Moreover, as can be seen on the last two columns, to minimize Jo the algorithm sometimes badly estimates the deformed object in order not to include a dark spot.
On the third line of Figure 2, we apply the model where the template is obtained from an ellipse hypertemplate but without any anomaly matrix (E8). This time, as expected, there is no dark shadow on the template but the reconstruction of the last two observations is still bad.
The model (E4) is also tested. To do so, a template from the 100 "control" observations is estimated. This template is then fixed in the minimization of equation (E4). This estimated template and some reconstructions are represented on the fourth line of Figure 2.
This time, the observations are better reconstructed and the dark spots retrieved. Their intensity is a bit weaker than initially due to the proximal gradient descent applying a soft threshold on the residuals. Moreover, the shapes of the dark spots are well identified and so is their position.
Then, the model E6 is applied where the template is estimated from a hypertemplate. The results are presented on the last line of Figure 2. Once again, the observations are well reconstructed and the dark spots are retrieved in the anomaly matrix. Shapes, positions and volumes of the dark spots are captured.
Finally, the four above mentioned models are compared by computing the mean error of registration when one only considers the form (i.e. the error between the observations without their dark spots and the reconstructions without their anomaly matrix) on table 1. As expected, the error is smaller when considering the models (E4) and (E6).

Table 1: Mean and standard deviation of the error of registration when one does not consider the dark spots.

|  | Model (E7) | Model (E8) | Model (E4) | Model (E6) |
|---|---|---|---|---|
| Error of registration | $19, 8\%_{\pm 006}$ | $16.5\%_{\pm 0.10}$ | $11.9\%_{\pm 0.07}$ | $12.9\%_{\pm 0.03}$ |

Hence, we have been able to reconstruct the observations and to retrieve their anomalies using the models (E4) and (E6). Moreover, an improvement of reconstruction has been highlighted when estimating both anomaly matrix and deformations.

The choice to estimate both the deformation v and the anomaly matrix A at the same time, and not one after the other, comes from an effort to improve the reconstruction of the object. Indeed, on Table 1, it can be seen that the errors of reconstruction are smaller when we estimate both at the same time. It can particularly be seen on the third line of Figure 2 where, for the last two columns, the residuals contain whole parts of the observations. Those parts would hence be retrieved in the anomaly matrix.

Example 2

This example aims emphasize this need to estimate both the deformation and the anomaly matrix at the same time. The different images of that example can be seen on Figure 3.

This time, a black line is added on the control template, mimicking for example a vein in a liver or a gyrus in a brain slice. From this template one observation is created to which a dark spot is added. We try to reconstruct this observation from the template, either with or without anomaly matrix.

Figure 3 shows, from left to right, the fixed template, the observation, the reconstruction without anomaly matrix and the reconstruction with anomaly matrix.

Without anomaly matrix, the model chooses to heavily deform the black line to create the dark spot. In particular, a part of the line would here be in the residual and the black spot would not entirely be in it. The estimation of an anomaly matrix from this residual would hence be bad. However, if both deformation and anomaly matrix are estimated at the same time, the black line is well registered from the template to the individual and only the black spot is retrieved in the anomaly matrix.

Those two observations confirm the need to couple the estimation of anomaly matrices and deformations.

Example 3

In this example, the model is applied to a data set of brains with tumors obtained from the BraTS 2018 data set (Bakas et al., 2017, 2018; Menze et al., 2014). More precisely, the data set is composed of 50 post-contrast T1-weighted MRI scans of glioblastoma and lower grade glioma.

We do not dispose of a data set of control subjects but only of the observation of one control subject. Hence, the model (E6) is used to estimate the template using this control subject as hypertemplate.

The goal is to reconstruct the brain of each subject from a control template and to obtain the tumors in the anomaly matrix. Ideally, the diffeomorphic deformation will register the brain folds (called gyri) and ventricles. What cannot be retrieved in the diffeomorphic deformation should hence only be the tumors.

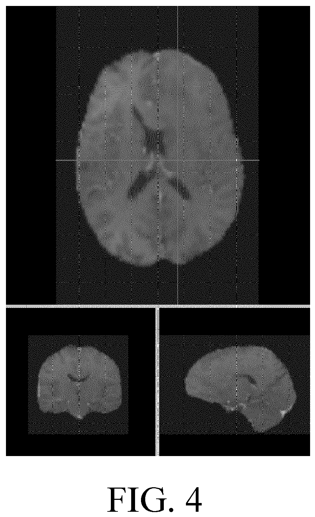

Figure 4 shows the template estimated by said algorithm.

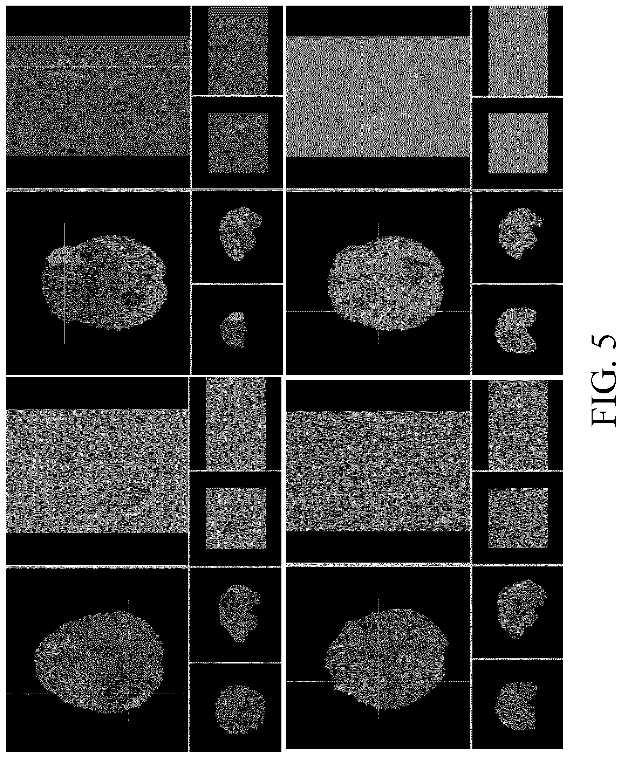

Figure 5 shows the results for observations of four different subjects. For each subject, said figure shows the corresponding observation on the left in three different planes and, on the right, the estimated anomaly matrix.

As can be seen, the lesions are retrieved in the anomaly matrix with only small errors of reconstruction. In particular, the gyri have been well registered and are not present in the anomaly matrix. As for the ventricles, if a part of one is present in the bottom right image, they are also well registered for the other images. In fact, for the bottom right patient, its right ventricle is quite different from the template, causing the algorithm difficulties to register a part of it. But, in all cases, the use of the LDDMM framework has allowed to register most of the parts of the brain without anomaly and so to obtain a clean anomaly matrix.

Here, the important choice of parameter in equation (E6) is in fact the sparsity constant $\lambda$. One could choose to take a smaller $\lambda$. This would allow to include the peritumoral edema (dark area around the tumor) in the anomaly matrix. However, we would then also include more reconstruction errors. Here, $\lambda$ has be chosen in order to visually detect the tumors but with as little reconstruction errors as possible, even if the whole tumor is not in the anomaly matrix. Such choice is sufficient to inform the doctors of possible anomalies, which are indeed included in the anomaly matrix.

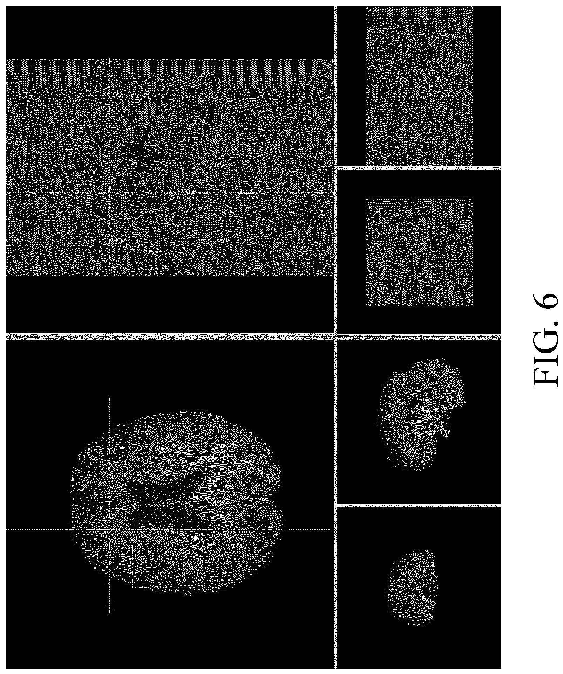

In fact, of the 62 tumors in the data set, 59 are visible in the anomaly matrix (95%). As for the tumors not visible, they are small lesions in a zone of high variability of the brain. Such a case is shown in Figure 6 where the tumor (boxed area) is less easy to distinguish and in the middle of the brain gyri.

Example 4

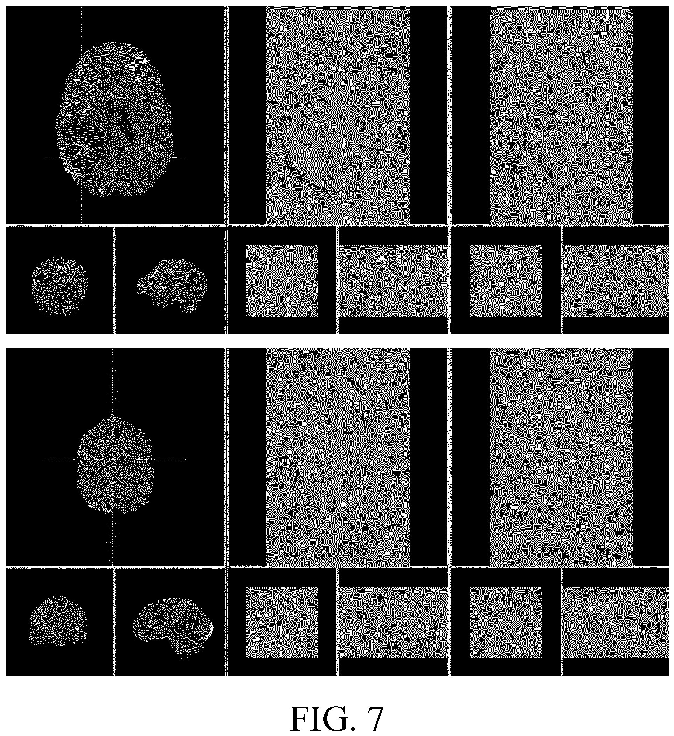

In this example, the approach does not require a large data set to be applied and there is no annotation on the position of the tumors required. To highlight this advantage, the exact same algorithm is applied to only one brain with tumors. As for the template, it is fixed as a brain without tumor. Hence, only two different brains are used to try to detect an anomaly. In particular, we compare the results with the anomaly matrix estimated for this patient in the previous section where a template was estimated alongside (see Figure 7).

Figure 7 shows the observation on left, the anomaly matrix estimated with the template fixed as a control subject on the center and, on the right, the anomaly matrix when one estimates the template, as done in example 3. The results are showed for two different slices: at the position of the tumor (top images) and at the top of the brain (bottom images). In both cases, the tumor is retrieved. More reconstruction errors are included when one does not estimate a template.

The tumor is once again retrieved in the anomaly matrix with small errors of reconstruction, particularly on the border and top of the brain. The errors on the top, in particular, are not present when one estimates a template alongside the anomaly matrix. In fact, the variability between the control subject and the one with tumor is bigger there, causing bigger errors of reconstruction. But, estimating a template, even with few subjects as done in example 3, prevents this issue.

Even if more errors are included in the sparse anomaly matrix, it must be emphasized that it has been produced using only two subjects and that it shows that the method according to the invention can yield usable results even without access to more than a few subjects.

Example 5

In this example, the algorithm of the invention is applied to a liver data set. The goal is to reconstruct the liver of each patient while recovering the tumors in the anomaly matrix. As we do not have access to a full data set of control patients, the model (E4) cannot be used and we need to estimate the template from a hypertemplate using model (E6). The hypertemplate is chosen as a patient or subject, not in the database, and without tumor.

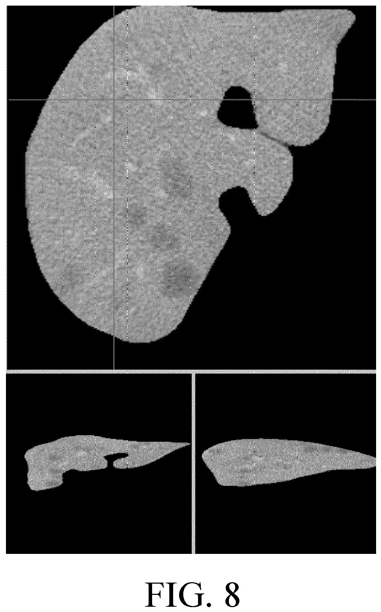

Two different structures appear on the livers: tumors as dark spots and vessels as white structures. Hence, it is those two structures we will recover in the anomaly matrix. If one only wants to retrieve the tumors, it is easy to separate them from the vessels according to their intensity. Moreover, from one subject to the other, the noise level can be totally different. If the data set is not preprocessed, we would not be able to find a sparsity constant $\lambda$ efficient for each subject and, for those with the highest level of noise, this noise would be recovered in the anomaly matrix. To prevent this phenomenon, we decide to first convolve the observations with a Gaussian kernel. The resulting images can be seen Figure 8.

Figure 8 shows, on the left, the initial images or observations and, on the right, the same images after convolution with said Gaussian kernel.

This smoothing allows to have a robust algorithm for this population. Moreover, because all images do not have the same pixel spacing, some of the images may be down sampled. We may also include all of them in a black box of the same size.

In the following, the results for different subjects are presented.

As a post process, the coefficients of the anomaly matrix to 0 outside of the reconstructions and targets. If one does not make this choice, the errors of reconstruction are reported in the anomaly matrix. In particular, one would find white zones in the anomaly matrix at voxels where the diffeomorphic deformation has not been able to recreate a liver part and black zones where the diffeomorphic deformation has created a liver part at a place there should not be one.

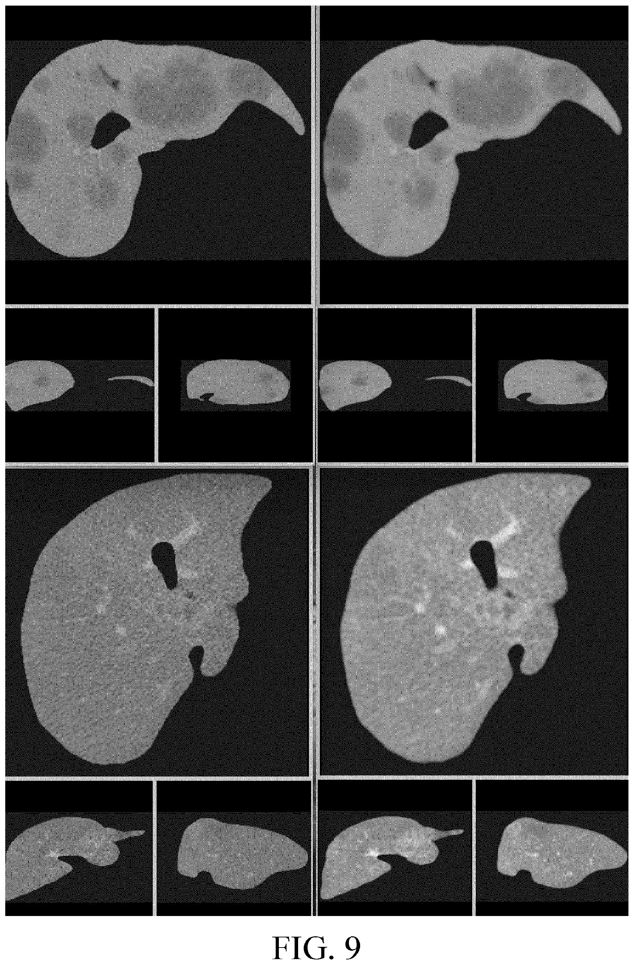

The template $\bar{y}$ estimated as a diffeomorphic deformation of a control patient is presented in figure 9. This estimation would particularly benefit from the use of a whole data set of control patients. Here, because it is derived from a particular control subject, the vessels of this particular subject are still present in the final template and can influence the future estimation of the anomaly matrix. Having a template of control subject would also surely reduce the errors of reconstruction and allow a better detection of the anomalies.

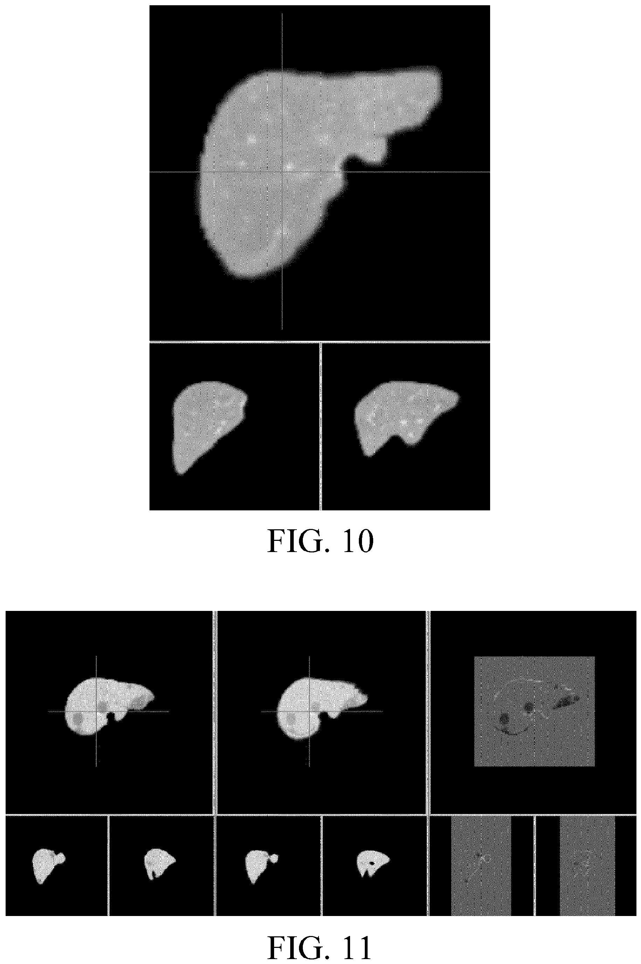

Then, Figure 10 shows the results for a subject without any vessel visible on the scanner.

In particular, in this figure, the observation is shown on the left (three different planes), the reconstruction is shown on the center and the anomaly matrix is shown on the right.

The algorithm according to the invention is able to retrieve the tumors in the anomaly matrix. The outline of the registration is also present in the anomaly matrix as it is used to obtain a better final reconstruction.

Figure 11 shows a subject with visible vessels. From left to right, this figure shows the observation, the anomaly matrix and the negative values of the anomaly matrix.

As can be seen, not only the tumors are retrieved but also the vessels. If one wants to only find the tumors, a first possibility is to look at the negative values of the anomaly matrix. Further investigation on a post process would be required to only retrieve the anomalies without the small errors of reconstruction.

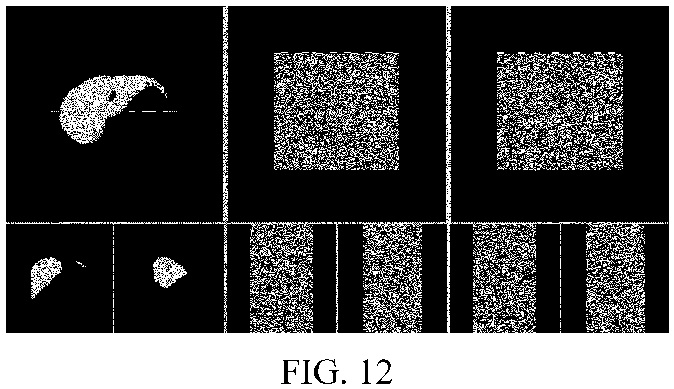

Finally, Figure 12 illustrates the importance to apply a Gaussian convolution to the data set before estimating the parameters of the model.

More particularly, figure 12 shows:

- on the top line, the results if the data set is not convolved with a Gaussian kernel beforehand;

- on the bottom, said results with preprocessing;
- on the left, the observation;
- in the center, the reconstruction;
- on the right, the anomaly matrix.

If one does not perform this preprocessing, the noise may be retrieved in the anomaly matrix and the tumors may not be visible. This problem is indeed solved after convolution and the tumor (at the top left of the liver) is retrieved.

To measure the quality of the detection, a MD Radiologist has segmented the tumors of 10 patients. This led to a total number of 133 tumors segmented. Here, we will only look at the negative coefficients of the sparse matrices to measure the quality of detection as tumors are dark spots on the liver.

We choose not to evaluate the segmentation but the detection. In fact, here, the dice score would be average as the algorithm rarely segments the whole tumor. However, here, the aim is not segment the exact tumor but only to inform the doctor of a possible anomaly and particularly detect very small lesions.

On the 133 tumors segmented, the algorithm according to the invention detects 125 of them (94%). As for the tumors which are not detected, there are two possibilities. Sometimes, the difference between the tumor intensity and the noise is really small and the algorithm is not able to separate them, in particular for some subjects for which the noise is still high. The other possibility is when the diffeomorphic registration of the liver is not perfect and the tumor is outside of it. In that case, the tumor will in fact be in the anomaly matrix but it will be lost in the error of reconstruction.

Finally, not only the tumors are retrieved in the anomaly matrix. As showed above, small errors of reconstruction can be present on the boundary. But the algorithm also plays its part by detecting other anomalies than lesions. In particular, on several subjects, some slightly dark spots are retrieved and are in fact due to a perfusion disorder.

Said example show that the residuals of the diffeomorphic deformation from a control template may be used to detect and segment lesions in an organ. Moreover, it appears that it can even improve the diffeomorphic reconstruction of the observations. This method has the advantage not to require a large data set of sick patients nor annotations from medical doctors. It is hence particularly suited to the detection of anomalies, in particular for specific treatment protocols where it is often impossible to obtain large data sets. The efficiency of this method is also shown on a data set of brains with glioma and on a data set of livers. In particular, in the former case, the method according to the invention has been able to register the gyri and the ventricles of the brains while retrieving the tumors in the anomaly matrix.

**Claims**

1. A method implemented by computer means for characterizing at least one observation y of a subject, comprising the steps of:

    - determining a template $\bar{y}$ characterizing a population of subjects without anomaly, by diffeomorphic deformation,
    - minimizing the cost function J :

$$J(F,A) = K\|y - F(\bar{y}) - A\|^2 + \lambda\|A\|_1 + Reg(F)$$

    where:

    F is a deformation function,
    $A$ is an anomaly matrix,
    $\|A\|_1$ is the 1-norm of A,
    K and $\lambda$ are predefined constants,
    Reg is a regularization function,

    where during minimization, F and A are determined by learning, F providing information on the morphological variability of the subject and A providing information on anomalies in said observation y.

2. The method according to claim 1, wherein said observation is an image.

3. The method according to claim 1, wherein said observation is a feature of an image, for example a contour.

4. The method according to claim 2 or 3, wherein said image is a medical image.

5. The method according to any of the preceding claims, wherein the template $\bar{y}$ is obtained using Large Diffeomorphic Deformation Metric Mapping.

6. The method according to any of the preceding claims, wherein the template $\bar{y}$ is based on a plurality of observations that do not contain anomalies.

7. The method according to any of the preceding claims, wherein the template $\bar{y}$ is based on only one observation that do not contain anomalies and at least one observation that does contain anomalies.

8. A computer software, comprising instructions to implement at least a part of the method according to any of the preceding claims when the software is executed by a processor.

9. Computer device (1) comprising:

   - an input interface (2) to receive at least one observation y of a subject,
   - a memory (3) for storing at least instructions of a computer program according to claim 8,
   - a processor (4) accessing to the memory (3) for reading the aforesaid instructions and executing then the method according to any of the claims 1 to 7,
   - an output interface (5) to provide F and A determined during minimization of the cost function $J$.

10. A computer-readable non-transient recording medium on which a computer software is registered to implement the method according to any of the claims 1 to 8, when the computer software is executed by a processor (4).

FIG. 1

FIG. 2

FIG. 3

FIG. 4

FIG. 5

FIG. 6

FIG. 7

FIG. 8

FIG. 9

FIG. 10

FIG. 11

FIG. 12

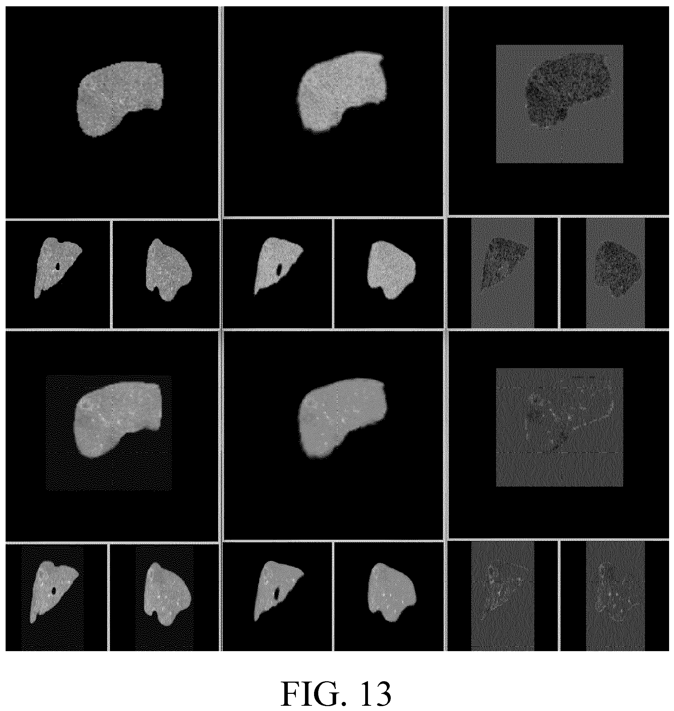

FIG. 13

**EP 4 296 951 A1**

## EUROPEAN SEARCH REPORT

Application Number

EP 22 30 5905

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DURRLEMAN STANLEY ET AL: "Morphometry of anatomical shape complexes with dense deformations and sparse parameters", NEUROIMAGE, ELSEVIER, AMSTERDAM, NL, vol. 101, 26 June 2014 (2014-06-26), pages 35-49, XP029054736, ISSN: 1053-8119, DOI: 10.1016/J.NEUROIMAGE.2014.06.043 * abstract * | 1-10 | INV.<br>G06T7/77<br>G06T7/00<br>G06T7/30 |
| A | MATTHIS MAILLARD ET AL: "A deep residual learning implementation of Metamorphosis", ARXIV.ORG, CORNELL UNIVERSITY LIBRARY, 201 OLIN LIBRARY CORNELL UNIVERSITY ITHACA, NY 14853, 1 February 2022 (2022-02-01), XP091148992, * abstract * | 1-10 | |
| A | ANUPAMA GOPARAJU ET AL: "Benchmarking off-the-shelf statistical shape modeling tools in clinical applications", ARXIV.ORG, CORNELL UNIVERSITY LIBRARY, 201 OLIN LIBRARY CORNELL UNIVERSITY ITHACA, NY 14853, 7 September 2020 (2020-09-07), XP081756300, * abstract * * section 3.1.2. Deformetrica * | 1-10 | TECHNICAL FIELDS SEARCHED (IPC)<br><br>G06T<br>G06V |

The present search report has been drawn up for all claims

| Place of search | Date of completion of the search | Examiner |
|---|---|---|
| Munich | 24 November 2022 | Rimassa, Simone |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.....................................................................
& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ALAIN TROUVE.** *An infinite dimensional group approach for physics based models in pattern recognition,* 1995
- **PAUL DUPUIS ; ULF GRENANDER ; MICHAEL I MILLER.** Variational problems on flows of diffeomorphisms for image matching. *Quarterly of applied mathematics,* 1998, 587-600
- **M FAISAL BEG ; MICHAEL I MILLER ; ALAIN TROUVE ; LAURENT YOUNES.** Computing large deformation metric mappings via geodesic flows of diffeomorphisms. *International journal of computer vision,* 2005, vol. 61 (2), 139-157
- **S. ALLASSONNIÈRE ; E. KUHN ; A. TROUVÉ.** Construction of Bayesian deformable models via stochastic approximation algorithm: A convergence study. *Bernoulli Journal,* 2010, vol. 16 (3), 641-678